# EUROPEAN PATENT APPLICATION

(11) **EP 2 716 638 A1**
(43) Date of publication of application: **09.04.2014**
(21) Application number: 12306226.7
(22) Date of filing: 08.10.2012
(51) Int. Cl.: C07D 403/12, A61K 31/4196, A61K 31/496

(54) **Labeled ligands of the oxytocyn receptor**

(71) Applicant: Université de Strasbourg, 67000 Strasbourg (FR); Centre National de la Recherche Scientifique (C.N.R.S), 75016 Paris (FR)
(72) Inventor: Dominique Bonnet, 67118 Geispolsheim (FR); Thierry Durroux, 34980 Saint Gely du Fesc (FR); Marcel Hibert, 67114 Eschau (FR)
(74) Representative: Nevant, Marc

(57) **Abstract**

The invention relates to compounds of formula: 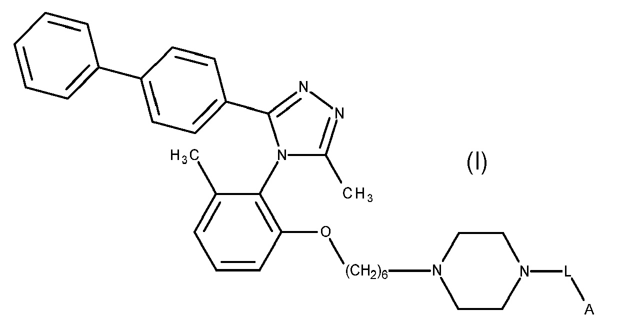 wherein L and A are as defined in the description.

These compounds can be used as ligands of the oxytocin receptor.

## Description

The present invention relates to non-peptidic labeled ligands of the oxytocin receptor (OTR) that do not specifically bind to the vasopressin receptor V2 (V2R); in particular, the invention relates to fluorescent ligands of OTR, as well as to a method of labeling OTR with these ligands.

### Background of the invention

There is growing evidence that the neuropeptides oxytocin (OT) and arginine vasopressin (AVP) modulate complex social behavior and social cognition. Oxytocin has a single identified receptor OTR, whereas vasopressin acts in the brain on its two centrally expressed receptor subtypes, V1a and V1b. Of these two receptors, V1a plays a more prominent role in vasopressinergic modulation of social behavior and thus has been the focus of most research examining vasopressin's role in regulating social behavior. Animal studies described the involvement of OT and AVP in many central nervous system (CNS) functions including mating, affiliation, adult pairing and faithfulness, anxiety, and prosocial behaviors. Human studies are beginning to explore the roles of these two neuropeptides in social cognition and behavior and suggest that variation in the genes encoding their receptors (OTR and V1aR, respectively) may contribute to variation in human social behavior by altering brain function. Recently, OT has been reported to improve trust and to decrease fear associated to social phobia and symptoms related to autism in human. To investigate these functions, the only molecular probe currently available is OT itself or closely related peptide analogues that have a poor bioavailability (poor blood-brain barrier penetration, short half-life, rapid clearance). In this context, there is a need for centrally active, potent, specific and bioavailable agonists/antagonists of OTR.

5-biphenyl-4-phenyl-1,2,4-triazole derivatives have been described as a class of selective antagonists for the human V1aR (Kakefuda et al. J Med Chem 2002, 45, 2589-98).

Fluorescence resonance energy transfer (FRET) between a fluorescent donor-acceptor pair has been shown to be a convenient method to investigate intra- and intermolecular interaction processes both *in vitro* and *in vivo.* Time-resolved fluorescence resonance energy transfer (TR-FRET), using rare-earth lanthanides with long emission half-lives as donor fluorophores, combines standard FRET with the time resolved measurement of fluorescence. This powerful combination provides significant benefits for HTS. Nevertheless, the prerequisite for the development of such fluorometric assays is to design and to synthesize fluorescent ligands that retain the pharmacological profile of the non-labeled probes.

There is a need for high-affinity non-peptidic fluorescent ligands for OTR, since such compounds are currently not available and would accelerate the discovery of novel small organic agonists/antagonists of OTR.

### Description of the drawings

Figure 1 represents the FRET signal obtained with fluorescent compounds of the invention as a function of the concentration of said compounds.
Figure 2 represents the FRET signal obtained with fluorescent compounds of the invention as a function of AVP or oxytocin concentrations.

### Description of the invention

The object of the invention is to provide compounds that specifically and selectively bind to the oxytocin receptor (hereafter OTR), as compared to the V2 receptor (V2R), and that can be easily conjugated with labels, particularly fluorescent labels.

The expression "specific and selective binding to the OTR as compared to V2R" means that the affinity constant of the compounds for OTR is 10 times higher, preferably 50 times higher and even more preferably 100 times higher than their affinity constant for V2R.

In a first aspect, the invention is directed to a compound of formula (I): 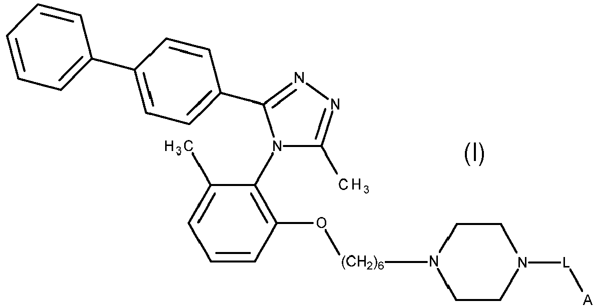 wherein L is a divalent linker and A is a functional group for coupling to a detectable labeling agent.

Preferably, L represents a divalent group chosen from: a substituted or unsubstituted, straight or branched chain, saturated or unsaturated C₁-C₁₀ alkylene moiety, preferably a C₂-C₆ alkylene moiety; a substituted or unsubstituted, saturated or unsaturated cycloalkylene moiety; a substituted or unsubstituted arylene moiety; a group of formula -CH₂CH₂(O-CH₂CH₂)ₙ-, wherein n is an integer having a value of 1 to 5. Suitable cycloalkylene moieties include cyclohexylene. Suitable arylene moieties include phenylene and xylylene.

Most preferably, L is a group of formula -CH₂CH₂(O-CH₂CH₂)ₙ- wherein n is as defined above, in particular n is 1, 2 or 3.

A is preferably a group chosen from: -NH₂, -NCS, -NCO, -N₃, -SH, -S-S-Ar or -NH-Ar, wherein Ar is a 5- or 6-membered, saturated or unsaturated heterocycle comprising 1 to 3 heteroatoms, said heterocycle being optionally substituted with a halogen atom, or A is chosen from one of the following groups :

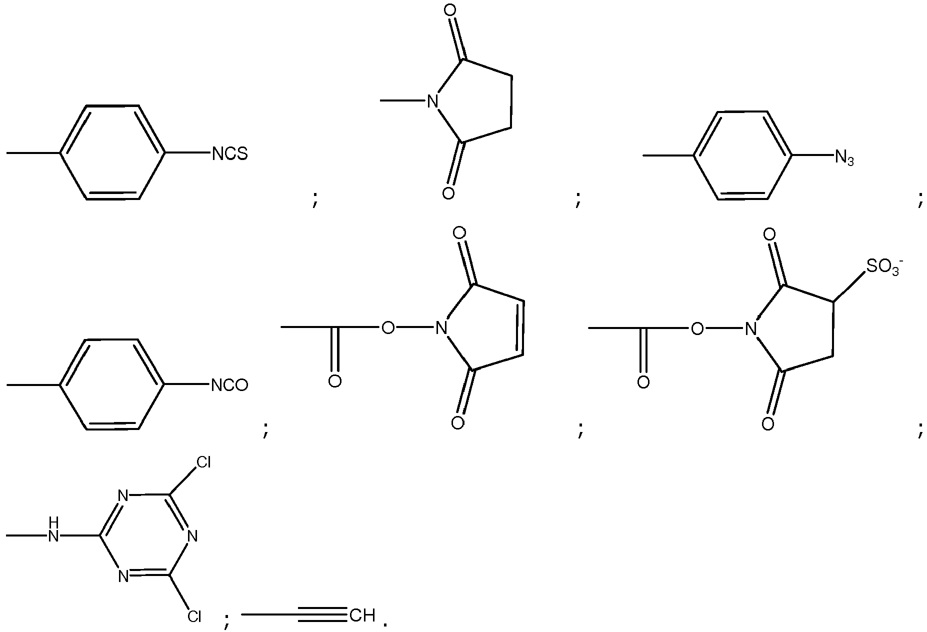

Examples of suitable Ar groups include the following groups: pyrrolyl, pyrazolyl, imidazolyl, oxazolyl, isoxazolyl, triazolyl, oxadiazolyl, furanyl, thienyl, thiazolyl, isothiazolyl, thiadiazolyl, pyridyl, pyridazinyl, pyrimidinyle pyrazinyl, triazinyl, pyrrolidinyl, tetrahydrofuryl, tetrahydrothienyl, pyrrolidinyl, imidazolidinyl, thiazolidinyl, isoxazolidinyl, piperidinyl, piperazinyl, morpholinyl, thiomorpholinyl.

Advantageously, A is -NH₂.

A preferred group of compounds include those where L is a group of formula -CH₂CH₂(O-CH₂CH₂)ₙ- wherein n is as defined above, and A is NH₂, these compounds are represented by formula (II):

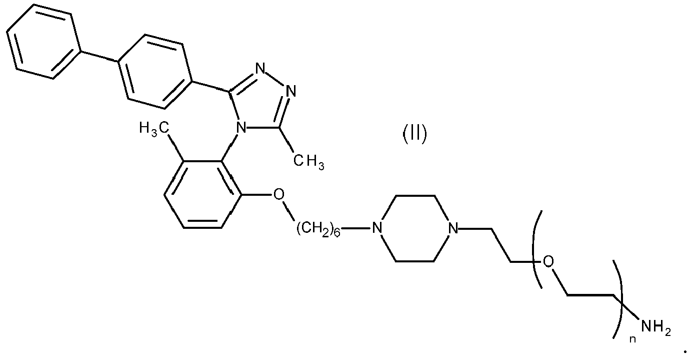

In another aspect the invention is directed to labeled compounds that are specific and selective to OTR as compared to V2R. Consequently, the invention also relates to a conjugate comprising a compound of the invention in which a detectable labeling agent is coupled to group A of said compound.

Preferably, the labeling agent is selected from an enzyme, a luminescent substance, or a radioactive substance.

More preferably, the labeling agent is a luminescent substance, which may be a bioluminescent, chemiluminescent or fluorescent material. Luminescent or enzymatic labeling agents are commercially available and are provided in a form suitable for conjugation with the compounds of the invention, i.e. they contain a functional group which will react with the functional group A of those compounds. The following labeling agents are particularly preferred since they are commonly used in biological analysis:
*Enzymatic labels:* horseradish peroxidase (HRP), luciferase. Those enzymes are commercially available in a form suitable for conjugation with the compounds of the invention.
*Fluorescent labels:* rhodamines, cyanines, squaraines, fluorophores known under the name BODIPY, fluoresceins, compounds known under the name AlexaFluor, rare-earth chelates, rare-earth cryptates, quantum dots, phycobiliproteins, such as B-phycoerythrin, R-phycoerythrin, C-phycocyanin, allophycocyanins. Again, those compounds are commercially available in a form that is suitable for conjugation with the compounds of the invention. Suitable fluorescent labels include Lumi4™Tb (available from Lumiphore Inc.) and Dy647 (available from Dyomics).

Alternatively, the labeling agent is a member of a pair of binding partners. Such binding partners may be one of the following pairs: avidin or streptavidin/biotin, DNP/anti-DNP antibodies. Consequently, the invention also relates to a conjugate of the M6G derivative of the invention, covalently bonded with biotin or DNP. Addition of the second member of the binding partner pair, itself labeled with a fluorescent or enzymatic tracer, will ensure the labeling of the compounds of the invention with a detectable compound.

It has been determined that the compounds of the invention can actually bind both OTR and V1aR with similar affinity. Consequently, another aspect of the invention relates to a method of studying OTR or V1aR potential ligands comprising contacting those potential ligands with OTR or V1aR in the presence of a labeled compound of the invention, and measuring the signal generated by said labeled compound. Comparison with the signal measured in the absence of the potential ligand or with different concentrations of the potential ligand will provide information as to the capacity of this potential ligand to bind with OTR or V1aR.

Another aspect of the invention relates to a method of labeling OTR or V1aR comprising contacting the labeled compounds of the invention with OTR or V1aR.

The invention is also directed to the use of a labeled compound according to the invention in a binding assay for the OTR or V1aR.

Finally, a last object of invention is the use of a labeled compound according to the invention to label OTR or V1aR.

### Preparation of the compounds and conjugates of the invention

The strategy developed for the synthesis of the labeled compounds of the invention involved the preparation of the triazole **10** (Scheme 1), of various activated PEG linkers **13-16** (Scheme 2), the condensation of the linkers onto triazole **10** followed by the final fluorescent dyes incorporation (Scheme 3). For the synthesis of triazole **10,** an original and convergent approach has been used, and its key step is the condensation of a thioamide with a hydrazide in the presence of a thiophile metal. Commercially available piperazine was monoprotected with *tert*-butyloxycarbonyl (Boc) moiety to yield 1-Boc piperazine **2** that was reacted with 6-bromohexan-1-ol to provide compound **3** in 48% yield (2 steps). In parallel, the acylation of 2-amino-3-methylphenol with acetic anhydride gave access to acetamide derivative **4** in 65% yield. The condensation of alcohols **3** and **4** was performed via a Mitsunobu reaction, for example in the presence of 1,1'-(azodicarbonyl)dipiperidine (ADDP) and tributylphosphine in THF, affording compound **5** in 79% yield. 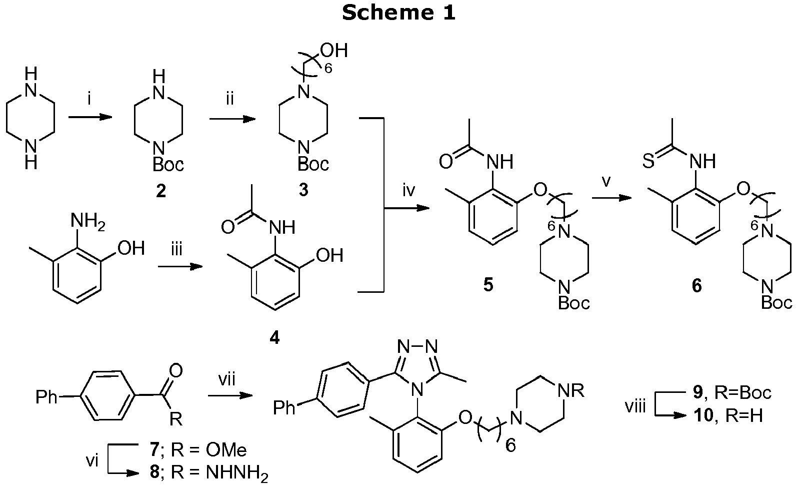 *Reagents used: (i) Boc₂O, CH₂Cl₂, (ii) K₂CO₃, ACN, reflux, (iii) Ac₂O, AcOEt, (iv) ADDP, PBu₃, THF, (v) Lawesson's reagent, toluene, reflux, (vi) hydrazine, EtOH, (vii) Hg(OAc)₂, DMF, (viii) TFA*/*CH₂Cl₂ 1*/*1*

The subsequent conversion of amide **5** in thioamide **6** was efficiently achieved in the presence of Lawesson's reagent in 83% yield. The hydrazinolysis of commercially available methyl 4-phenylbenzoate **7** yielded hydrazide **8** (72%) that was reacted with thioamide **6** in the presence of mercury (II) acetate, leading to the formation of triazole **9** in 37% yield. A subsequent treatment in acidic conditions enabled the Boc removal to yield triazole **10,** ready to be modified with various N-Boc protected PEG derivatives. As depicted in Scheme 2, these derivatives were obtained in three steps from commercially available diethyleneglycol and tetraethylene glycol. Free alcohols were activated as ditosylated derivatives **11** and **12** with tosyl chloride in basic conditions (62% and 67% yield, respectively). The monosubstitution of one tosyl group was achieved by slow addition of di-tert-butyl iminodicarboxylate into an excess of activated PEG derivatives at 0°C, leading to monoactivated derivatives **13** and **14** in 53% and 67% yield, respectively. A subsequent treatment with LiBr in DMF enabled the clean conversion of the tosylated derivatives into brominated derivatives **15** and **16** (83% and 71% yield, respectively).

The incorporation of these derivatives on triazole **10** was first achieved by nucleophilic substitution of tosylated derivatives **13** and **14,** providing compounds **17** and **18** in 40% and 17% yield, respectively (Scheme 3). Noteworthy, isolated yields were significantly improved by reacting triazole **10** with brominated derivatives **15** and **16** (61% and 46% yield, respectively). Compounds **17** and **18** were treated in the presence of TFA to provide compounds **19** and **20** in quantitative yields, which were subsequently acylated with the succinimic ester of DY647 or Lumi4-Tb to lead to fluorescent compounds **21-24** (Scheme 3). 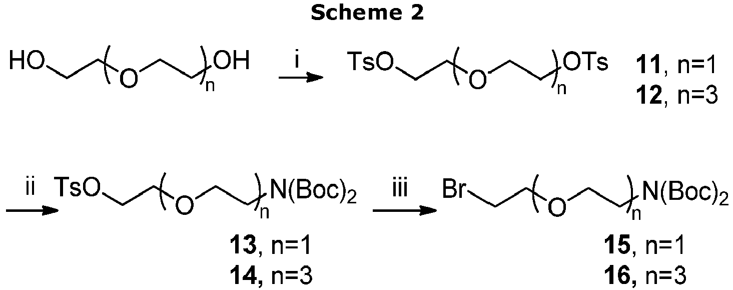 *Reagents used: (i) TsCl, Et₃N, CH₂Cl₂, (ii) NH(Boc)₂, Cs₂CO₃, DMF, (iii) LiBr, DMF.* 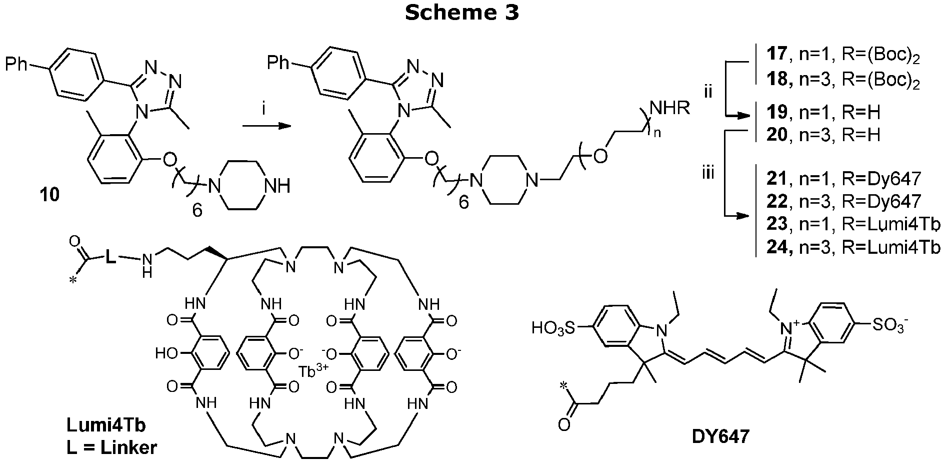 *Reagents used: (i) 15 or 16, Et₃N, CH₃CN, (ii) TFA*/*CH₂Cl₂, (iii) RCONHS, DIEA, DMSO.*

The invention will be illustrated by the examples below in which the following abbreviations are used.
CDCl₃ = deuterated chloroform
CH₃OD = monodeuteromethanol
CD₃OD = tetradeuteromethanol
CH₂Cl₂ = dichloromethane
DMF = N,N-dimethylformamide
DMSO = dimethylsulfoxide
eq. = equivalent
h = hour
HPLC = high-performance liquid chromatography
min = minute
NMR = nuclear magnetic resonance
quant. = quantitative
TFA = trifluoroacetic acid
THF = tetrahydrofuran
TMS = tetramethylsilane

Reagents were obtained from commercial sources and used without any further purification. Lumi4-Tb-NHS, SNAP-Lumi4-Tb, SNAP-RED and cAMP dynamic kit were purchased from Cisbio Bioassays (Codolet, France) and DY647-NHS from Dyomics GmbH (Jena, Germany). Thin-layer chromatography was performed on silica gel 60F₂₅₄ plates. Flash chromatography was performed on silica gel (40 µm, Grace) prepacked columns on a SPOT II ultima from Armen. Analytical reverse-phase high performance liquid chromatography (RP-HPLC) separations were performed on a C18 Agilent Eclipse XDB (5 µm, 4.6 mm x 150 mm) using a linear gradient (5% to 95% of solvent B in solvent A in 7 min, flow rate of 1.6 mL·min⁻¹, detection at 220 nm, solvent A: water/0.1%TFA, solvent B: acetonitrile/0.1% TFA). Purified final compounds eluted as single and symmetrical peaks (thereby confirming a purity of ≥95%) at retention times (*t*_{R}) given below. Their identity was determined by high resolution mass spectra (HRMS) which were acquired on a Bruker MicroTof mass spectrometer, using electrospray ionization (ESI) and a time-of-flight analyzer (TOF). ¹H and ¹³C spectra were recorded in DMSO-d6, CH₃OD, CD₃OD or CDCl₃ on Bruker Avance III 400 MHz spectrometer. Chemical shifts are reported as δ values in parts per million relative to TMS as an internal standard. Splitting pattern abbreviations are as follows: s = singlet, d = doublet, dd = double doublet, q = quartet, t = triplet, m = multiplet. Coupling constants are in Hertz. Analytical reverse-phase high performance liquid chromatography (RP-HPLC) separations were performed on a C18 Agilent Eclipse XDB (5 µm, 4.6 mm x 150 mm) using a linear gradient (5% to 95% of solvent B in solvent A in 7 min, flow rate of 1.6 mL·min⁻¹, detection at 220 nm, solvent A: water/0.1%TFA, solvent B: acetonitrile/0.1% TFA). Purified final compounds eluted as single and symmetrical peaks (thereby confirming a purity of ≥95%) at retention times (*t*_{R}) given below. Their identity was determined by high resolution mass spectra (HRMS) which were acquired on a Bruker MicroTof mass spectrometer, using electrospray ionization (ESI) and a time-of-flight analyzer (TOF).

### Example 1

### tert-butyl 4-(6-(2-acetamido-3-methylphenoxy)hexyl)piperazine-1-carboxylate (5)

To a stirred solution of 1,1'-(azodicarbonyl)dipiperidine (560 mg, 2.22 mmol) dissolved in anhydrous THF (17 mL) under an atmosphere of argon at 0 °C, was added tributylphosphine (555 µL, 2.22 mmol). The reaction mixture was stirred at room temperature for 20 min and compound **3** (530 mg, 1.85 mmol) dissolved in anhydrous THF was added to the mixture. After 10 min, compound **4** (122 mg, 0.740 mmol) dissolved in anhydrous THF (3 mL) was added. The reaction mixture was stirred 1 h at 0 °C and overnight at room temperature. The solvent was concentrated *in vacuo.* Reverse-phase flash chromatography afforded the title compound **5** as a yellow oil (256 mg, 79%). *t*_{R} = 8.25 min. ¹H NMR (400 MHz, (DMSO-d₆): *δ* 7.07 (dd, 1H, *J* = 8.7, 7.4 Hz), 6.81 (s, 1H), 6.69 (dd, 1H, *J* = 8.1 Hz), 3.92 (dd, 2H, *J* = 8.1, 5.3 Hz), 3.45-3.39 (m, 4H), 2.39-2.31 (m, 6H), 2.21 (s, 3H), 2.16 (s, 3H), 1.75 (q, 4H, *J* = 6.9 Hz), 1.53-1.47 (m, 2H), 1.43 (s, 9H), 1.37-1.33 (m, 2H); ¹³C NMR (400 MHz, (DMSO-d₆): *δ* 168.97, 154.72, 152.94, 136.91, 127.20, 124.45, 122.66, 109.37, 79.65, 68.37, 58.60, 53.04, 44.08, 29.13, 28.44, 27.23, 26.00, 23.84, 18.55, 13.65.

### tert-butyl 4-(6-(2-ethanethioamido-3-methylphenoxy)hexyl)piperazine-1-carboxylate (6).

Compound **5** (450 mg, 1.038 mmol) was mixed with anhydrous toluene (14 mL) under an atmosphere of argon. Lawesson's reagent (335 mg, 0.830 mmol) was added and the reaction mixture was refluxed for 1h15. The solvent was concentrated *in vacuo.* Reverse-phase flash chromatography afforded the title compound **6** as a white powder (385 mg, 83%). *t*_{R} = 9.5 min. ¹H NMR (400 MHz, DMSO-d₆): *δ* 7.16 (dt, 1H, *J* = 14.4, 7.1 Hz), 6.81 (t, 1H, *J* = 7.3 Hz), 6.72 (t, 1H, *J* = 6.7 Hz), 3.87 (s, 2H), 3.79-3.70 (m, 4H), 3.56-2.90 (m, 6H), 2.67 (s, 2H), 2.21 (d, 3H, *J* = 5.3 Hz), 2.16 (s, 2H), 1.86-1.55 (m, 4H), 1.43 (s, 9H), 1.27 (s, 3H); ¹³C NMR (400 MHz, (DMSO-d₆): *δ* 202.28, 153.94, 153.83, 128.59, 126.39, 122.52, 112.97, 110.29, 80.86, 68.46, 57.10, 55.23, 51.52, 33.73, 29.52, 28.33, 26.21, 25.47, 17.98.

### tert-butyl4-(6-(2-(3-([1,1'-biphenyl]-4-yl)-5-methyl-4H-1,2,4-triazol-4-yl)-3-methylphenoxy)hexyl)piperazine-1-carboxylate (9)

To a stirred solution of compound **8** (141.61 mg, 0.667 mmol) and compound **6** (150 mg, 0.333 mmol) in anhydrous DMF (3.5 mL) was added mercury (II) acetate. The reaction mixture was stirred 2 days at room temperature. The mixture was filtered on celite (washing with CH₂Cl₂ and CH₂Cl₂/MeOH 1/1) and concentrated *in vacuo.* Reverse-phase flash chromatography afforded the title compound **9** as a clear oil (60 mg, 37%). *t*_{R} = 10.75 min. ¹H NMR (400 MHz, CD₃OD): δ 7.64-7.56 (m, 3H) 7.52-7.39 (m, 4H) 7.38-7.31 (m, 1H), 7.13-7.07 (m, 1H), 7.03-6.97 (m, 1H), 4.12- 3.92 (m, 2H), 3.45-3.40 (m, 1H), 3.37-3.35 (m, 3H), 3.32-3.29 (m, 3H), 2.48-2.35 (m, 2H), 2.27 (t, 3H, *J*= 5.4 Hz), 2.22 (s, 2H), 2.2-2.11 (m, 3H), 1.94 (s, 2H), 1.67-1.59 (m, 2H), 1.45 (d, 9H, *J* = 2.6 Hz), 1.39-1.33 (m, 2H), 1.31-1.27 (m, 1H), 1.27-1.14 (m, 3H); ¹³C NMR (400 MHz, CD₃OD): *δ* 156.45, 156.12, 155.05, 144.41, 141.07, 138.32, 132.88, 130.20, 129.23, 128.91, 128.42, 128.12, 124.16, 123.32, 112.07, 81.38, 69.73, 59.55, 54.14, 54.03, 48.51, 30.04, 28.15, 27.47, 27.05, 17.50.

### 1-(6-(2-(3-([1,1'-biphenyl]-4-yl)-5-methyl-4H-1,2,4-triazol-4-yl)-3-methylphenoxy)hexyl)pipérazine (10)

Compound **9** was dissolved in CH₂Cl₂/TFA (1/1, v/v, 6 mL). The mixture was stirred during 2h30, concentrated *in vacuo* and freeze-dried to afford the title compound **10** as a white powder (quant., 95%). *t*_{R} = 8.37 min. ¹H NMR (400 MHz, CDCl₃): δ 7.61-7.3 (m, CH), 6.96 (t, 2H, *J* = 8.8 Hz), 4.02-3.87 (m, 2H), 3.63 (s, 8H), 2.99 (s, 2H), 2.39 (s, 3H), 1.97 (s, 3H), 1.7-1.5 (m, 4H), 1.27-1.1 (m, 4H); ¹³C NMR (400 MHz, CDCl₃): δ 154.19, 144.81, 139.26, 136.81, 132.97, 129.23, 128.64, 128.04, 127.95, 127.24, 123.67, 111.07 , 69.01, 57.15, 48.36, 41.02, 28.49, 25.95, 25.43, 23.33, 17.48.

Compounds **17** and **18** were prepared according to the following procedure. To a stirred solution of compound **10** (1 eq.) dissolved in anhydrous CH₃CN was added triethylamine (5 eq.) and either compound **15** or compound **16** (3 eq.). The reaction mixture was stirred 48 h at room temperature and concentrated *in vacuo.* Compounds **17** and **18** were isolated by reverse-phase HPLC (Waters Symmetry Shield RP-C₁₈ column, 7 µm, 19 x 300 mm; linear gradient of solvent B in solvent A, solvent A: water/0.1% trifluoroacetic acid (TFA); solvent B: acetonitrile/0.1% TFA; flow rate of 20 mL·min⁻¹; detection at 254 nm).

### 2-(2-(4-(6-(2-(3-([1,1'-biphenyl]-4-yl)-5-methyl-4H-1,2,4-triazol-4-yl)-3-methylphenoxy)hexyl)piperazin-1-yl)ethoxy)ethanamine (17)

Yellow oil (14 mg, 31%). *t*_{R} = 5.02 min. ¹H NMR (400 MHz, CDCl₃): *δ* = 7.55-7.50 (m, 4H), 7.41-7.36 (m, 3H, CH aro), 7.34-7.32 (m, 2H, CH aro), 7.28-7.25 (m, 1H, CH aro), 7.01 (d, 1H, *J* = 8.8 Hz, CH aro), 6.92 (d, 1H, *J* = 8.8 ,CH aro), 4.01-3.91 (m, 2H, CH₂), 3.70 (t, 2H, *J* = 6Hz, CH₂), 3.61 (t, 2H, *J* = 5.2 Hz, CH₂), 3.51 (t, 2H, *J* = 5.7 Hz, CH₂), 3.13 (m, 3H, CH₃), 3.02 (s, 3H, CH₃), 2.88 (t, 2H, *J* = 5.1 Hz, CH₂), 2.78 (t, 2H, CH₂), 2.16 (s, 3H, CH₃), 1.87 (s, 3H, CH₃), 1.59-1.53 (m, 2H, CH₂), 1.40 (s, 18H, CH₃), 1.22-1.19 (m, 4H, CH₂). ¹³C NMR (100 MHz, CDCl₃): δ = 154.27, 144.63, 138.11, 132.92, 130.16, 129.23, 128.91, 128.44, 128.01, 124.22, 123.00, 112.01, 83.93, 70.22, 69.87, 68.02, 57.86, 51.63, 46.33, 29.72, 28.49, 27.22, 26.86, 25.31, 17.32, 10.41. MS (ESI/MeOH) *mlz:* 797.4 (M+H)⁺.

### 2-(2-(2-(2-(4-(6-(2-(3-([1,1'-biphenyl]-4-yl)-5-methyl-4H-1,2,4-triazol-4-yl)-3-methylphenoxy)hexyl)piperazin-1-yl)ethoxy)ethoxy)ethoxy)-ethanamine (18)

Clear oil (16 mg, 46%). *t*_{R} =5.16 min. ¹H RMN (400 MHz, CH₃OD): δ = 7.57-7.50 (m, 4H, CH aro), 7.43-7.33 (m, 5H, CH aro), 7.29-7.25 (m, 1H, CH aro), 7.03 (d, 1H, *J* = 8.5 Hz, CH aro), 6.94 (d, 1H, *J* = 8.5 Hz, CH aro), 4.00-3.92 (m, 2H, CH₂), 3.76-3.74 (m, 2H, CH₂), 3.72-3.69 (m, 2H, CH₂), 3.58-3.53 (m, 8H, CH₂), 3.49-3.47 (m, 4H, CH₂), 3.34-3.32 (m, 2H, CH₂), 3.00-2.96 (m, 2H, CH₂), 2.89 (s, 3H, CH₃) 2.76 (s, 3H, CH₃), 2.22 (s, 4H, CH₂), 1.90 (s, 4H, CH₂), 1.57-1.52 (m, 2H, CH₂), 1.40 (s, 18H, CH₃), 1.21-1.16 (m, 4H, CH₂). ¹³C RMN (100 MHz, CH₃OD): δ = 161.67, 151.20, 138.21, 133.32, 130.16, 129.04, 128.53, 128.05, 125.54, 124.33, 112.15 , 84.04, 78.99, 71.51, 71.33, 69.89, 66.00, 61.08, 57.74, 57.39, 55.33, 50.51, 49.46, 38.13, 31.67, 29.65, 28.81, 28.38, 27.10, 26.63, 24.92, 17.36, 10.35. MS (ESI/MeOH) *m*/*z:* 885.4 (M+H)⁺.

Compounds **19** and **20** were prepared according to the following procedure. Compounds **17** and **18** were dissolved in a TFA/dichloromethane mixture (1/1) and stirred for 3 h at room temperature. The mixture was concentrated *in vacuo* and freeze-dried.

### (2-((4-(6-(2-(3-([1,1'-biphenyl]-4-yl)-5-methyl-4H-1,2,4-triazol-4-yl)-3-methylphenoxy)hexyl)piperazin-1-yl)methoxy)ethanamine (19).

Clear oil (8.0 mg, 76%). *t*_{R} = 4.13 min. ¹H NMR (500 MHz, CD₃OD): *δ* 7.58-7.66 (m, 4H), 7.47-7.52 (m, 3H), 7.43 (t, 2H, *J* = 7.5 Hz), 7.36 (t, 1H, *J* = 7.5 Hz), 7.11 (d, 1H, *J* = 8.2 Hz), 7.02 (d, 1H, *J* = 8.2 Hz), 3.98-4.11 (m, 2H), 3.81 (t, 2H, *J* = 4.9 Hz), 3.73 (t, 2H, *J* = 4.9 Hz), 3.37-3.58 (m, 5H), 3.14-3.28 (m, 4H), 2.99-3.28 (m, 1H), 2.28 (s, 3H), 1.97 (s, 4H), 1.54-1.77 (m, 5H), 1.19-1.42 (m, 6H). ¹³C NMR (125 MHz, CD₃OD): δ 155.9, 144.8, 140.9, 138.3, 133.2, 130.2, 129.3, 129.0, 128.5, 128.1, 126.2" 124.3, 112.2, 69.9, 68.1, 66.7, 57.8, 57.4, 54.9, 50.8, 50.7, 40.5, 29.7, 27.2, 26.7, 25.0, 17.5, 10.5. MS (ESI/MeOH) *m*/*z* : 574.40102 (M+H)⁺

### 2-(2-(2-((4-(6-(2-(3-([1,1'-biphenyl]-4-yl)-5-methyl-4H-1,2,4-triazol-4-yl)-3-methylphenoxy)hexyl)piperazin-1-yl)methoxy)ethoxy)ethoxy)ethylamine (20).

Clear oil (11 mg, 88%). t_{R} = 4.52 min. ¹H NMR (400 MHz, CD₃OD): *δ* 7.64 (dd, 4H, *J* = 18, 8.2 Hz), 7.54-7.42 (m, 5H), 7.40-7.34 (m, 1H), 7.13 (d, 1H, *J* = 8.4 Hz), 7.04 (d, 1H, *J* = 7.7 Hz), 4.11-4.00 (m, 2H), 3.87-3.83 (m, 2H), 3.72-3.53 (m, 18H), 3.42-3.37 (m, 2H), 3.13 (t, 2H, *J* = 5 Hz), 3.10-3.04 (m, 2H), 2.32 (s, 3H), 2.00 (s, 3H), 1.69-1.57 (m, 4H), 1.32-1.22 (m, 4H); ¹³C NMR (400 MHz, CD₃OD): *δ* 162.26, 156.10, 145.13, 140.62, 138.23, 135.83, 133.36, 130.15, 129.36, 129.06, 128.54, 128.06, 124.34, 112.15, 71.56, 71.50, 71.44, 69.99, 68.01, 66.39, 57.81, 57.42, 50.59, 50.16, 40.74, 29.74, 29.75, 27.18, 26.70, 24.92, 17.46, 10.42. MS (ESI/MeOH) *m*/*z :* 685.44063 (M+H)⁺.

Compounds **19** and **20** were labelled with DY647 according to the following procedure. To a solution of DY647-N-hydrosuccinimidyl ester (3.8 µmol) in dry DMSO (325 µL) were added in one portion compound **19** or **20** (3.5 µmol) and diisopropylethylamine (5 µL, 26.6 µmol). The mixture was stirred at room temperature for 2 h. The mixture was purified by preparative RP-HPLC (Waters XBridge RP-C₁₈ column, 5 µm, 19 x 100 mm; 5 to 60% of solvent B in solvent A, solvent A: water/trifluoroacetic acid (TFA) 0.2%; solvent B: acetonitrile/0.2% TFA; flow rate of 20 mL·min⁻¹; detection at 280 nm) to give fluorescent probes **21** and **22.**

**DY647 labeled compound 21.** Blue powder (725 nmol, 21%). UV (water): λmax 650 nm. RP-HPLC purity: >95%; *t*_{R} = 16.41 min. HR-MS for C₆₈H₈₄N₈O₉S₂: calcd 1220.5803, found 1220.5783.

**DY647 labeled compound 22.** Blue powder (1008 nmol, 31%). UV (water): λmax 650 nm. RP-HPLC purity: >95%; *t_{R}* = 16.18 min. HR-MS for C₇₂H₉₂N₈O₁₁S₂: calcd 1308.6327, found 1308.6320.

Compounds **19** and **20** were also labelled with Lumi4-Tb according to the following procedure. To a solution of Lumi4-Tb N-hydrosuccinimidyl ester (3.8 µmol) in dry DMSO (362 µL) were added in one portion compound **19** or **20** (3.5 µmol) and diisopropylethylamine (8 µL, 26.6 µmol). The mixture was stirred at room temperature for 1 h. The mixture was purified by preparative RP-HPLC (Waters XBridge RP-C₁₈ column, 5 µm, 19 x 100 mm; 5 to 60% of solvent B in solvent A, solvent A: 25 mM triethylammonium acetate buffer (pH 6); solvent B: acetonitrile; flow rate of 20 mL.min⁻¹; detection at 280 nm) to give fluorescent probes **23** and **24.**

**Lumi4-Tb labeled compound 23.** White powder (770 nmol, 22%). UV (water): Imax 339 nm. RP-HPLC purity: >95%; *t_{R}* = 14.89 min. HR-MS for C₁₀₀H₁₂₈N₁₉O₁₆Tb: calcd 2009.9040, found 2009.9027.

**Lumi4-Tb labeled compound 24.** White powder (708 nmol, 20%). UV (water): Imax 339 nm. RP-HPLC purity: >95%; *t_{R}* = 15.02 min. HR-MS for C₁₀₄H₁₃₆N₁₉O₁₈Tb: calcd 2097.9564, found 2097.9538.

### Example 2

### Fluorescent ligand-binding assays

These assays were performed as described in Zwier et al (Journal of Biomolecular Screening 2010, 15, 1248-1259). Briefly, HEK 293T cells were cultivated in DMEM medium supplemented with 10% foetal calf serum (FCS) and 1% penicillin/streptomycin antibiotics. Confluent cells were dissociated and electroporated with plasmid DNA coding for the V2 receptor fused to the SNAP-tag suicide enzyme. Transfected cells were then plated in black 96-well plates previously coated with poly-ornithine (diluted at 0.1 mg.mL⁻¹ in sterile water, incubated 30 min at 37°C, washed with sterile PBS). Plates were incubated in supplemented DMEM medium for 48h à 37°C, 95% O₂ and 5% CO₂. Expressed receptors fused to SNAPtag were then labeled with fluorescent substrates, either with a donor (SNAP-Lumi4-Tb) or an acceptor (SNAP-RED) at respectively 100 or 300 nM in 100 µL of Tag-lite® labeling medium, for 1 h at 37 °C. Cells were then washed 3 times in Tag-lite medium and then incubated with the fluorescent ligands. For saturation assays, increasing concentrations of fluorescent ligands were diluted in 100 µL of Tag-lite medium. For competition assays, a fixed concentration of fluorescent ligand (typically 20 nM) was mixed with an increasing concentration of cold competitor ligand (in this case AVP) in Tag-lite medium. After overnight incubation at 4 °C (to prevent any internalization phenomenon), plates were read in an HTRF®-compatible multi-well plate reader with a classic HTRF® protocol (excitation at 340 nM, donor emission measured at 620 nm and acceptor emission at 665 nm, 50 µs delay, 500 µs integration).

### Fluorescent based-assay for V1aR and OTR

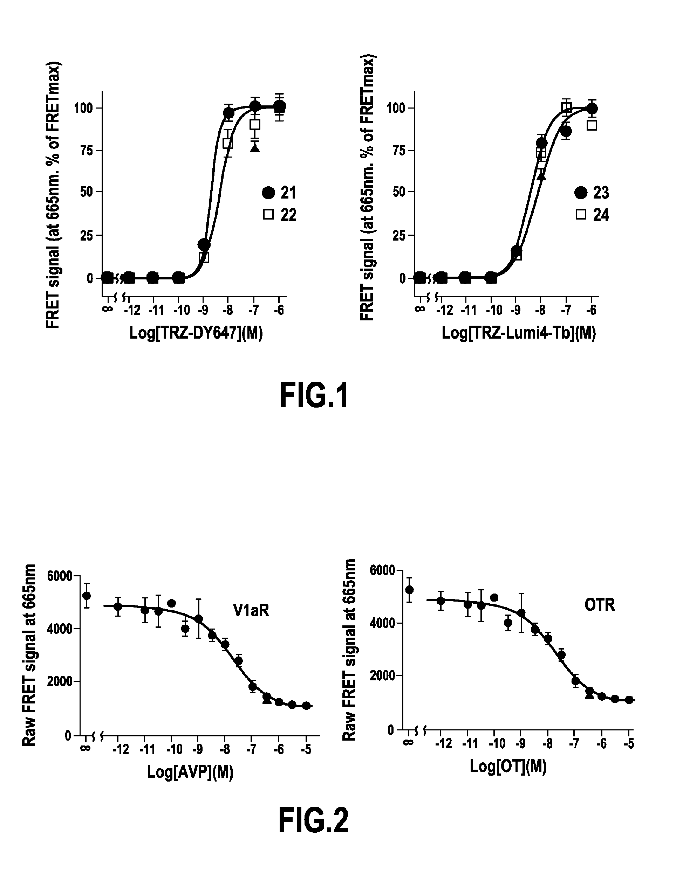

Fluorescent compounds **21** to **24** were involved in the development of a TR-FRET based assay for both V1aR and OTR. Such technique usually offers a higher signal-to-noise ratio than classic FRET because of the long-lasting fluorescence of the lanthanide cryptate. TR-FRET based saturation experiments with fluorescent compounds **21** to **24** were performed on V1a, OT and V2 receptors labeled with the SNAP-tag® approach: cells expressing a receptor of interest fused to SNAP-tag are incubated in the presence of a fluorescent substrate to label the receptor. Cells were then incubated in the presence of increasing concentrations of a fluorescent ligand. Fluorophores of the SNAP-tag substrates and of the ligands are chosen to be compatible with time-resolved FRET. SNAP-tagged receptors were labeled either with a Lumi4-Tb donor (SNAP-Lumi4-Tb) or d2 acceptor (SNAP-red) depending on the nature of the ligands, either acceptor **(21, 22)** or donor **(23, 24).** Saturation curves were obtained when plotting specific FRET signal as a function of fluorescent compounds concentration; non-specific binding was determined in the presence of an excess of the unlabeled corresponding ligands (10 µM) (Figure 1).

As shown in Table 1, the resulting dissociation constants (*K*d) determined for compounds **21** to **24** were found to be in the nanomolar range on V1aR (4-10 nM) and close to the *K*i determined in the literature for compound **1** (1.46 nM). The addition of bulky dyes such as DY647 or Lumi4Tb was not detrimental for the affinity for V1aR, regardless of the length of the linker. In addition, the same experiments performed on OTR showed that all the tested compounds display affinities close to those determined for V1aR. Thus, the fluorescent compounds of the invention enable TR-FRET based assays development on both V1aR and OTR.

Table 1 also shows the functional activity of fluorescent compounds **21** and **23** evaluated by the measurement of cyclic adenosine monophosphate (cAMP) accumulation following cell incubation with fluorescent compound at a concentration of 100-fold *K*i. No significant increase in accumulation of second messengers was detected for both ligands, whereas AVP displayed full agonist properties and a high potency (*K*act = 0.29 nM). This result indicates that compounds **21** and **23** are devoid of intrinsic agonist activity. Moreover, they fully inhibited AVP-induced cAMP accumulation in a dose-dependent manner, highlighting their antagonistic activity.

**Table 1**

| | | | | | |
|---|---|---|---|---|---|
| 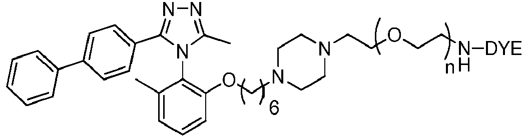 | | | | | |

| **Cpd** | **n** | **DYE** | **Binding, HTRF^{a} (K_{d}, nM)** | | |
|---|---|---|---|---|---|
| | | | **V₁ₐ** | **OT** | **V₂** |
| **21** | 1 | DY647 | 6 ±2 | 7 ± 2 | >1000 |
| **22** | 3 | DY647 | 10 ± 2 | 11 ± 2 | >1000 |
| **23** | 1 | Lumi₄Tb | 4 ± 1 | 3 ± 1 | >1000 |
| **24** | 3 | Lumi₄Tb | 4 ± 1 | 5 ± 4 | >1000 |

| | | | | | |
|---|---|---|---|---|---|
| *Results expressed as mean* ± *SEM of three independent* *separate experiments performed in triplicate* | | | | | |

To validate further the pharmacological assay, fluorescent compounds **21** (acceptor) and **23** (donor) were evaluated in competition experiments with either AVP or OT as competitor. Cells expressing Vasopressin V1a or OT receptors were labeled using the SNAP-tag technology and were incubated in the presence of compound **21** (20 nM) and increasing concentration of AVP or OT. Fluorescence intensity measured at 665 nm was plotted as a function of AVP or oxytocin concentrations. The Ki were determined with Cheng-Prussof equation. Competition curves obtained with compound **21** on V1aR and OTR are shown in Figure 2. Inhibition constants (*K*i) for AVP and OT determined by such competition assays with compound **21** are in agreement with their Kd determined by saturation with [³H]AVP. Similar results were obtained with compound **23,** validating that both fluorescent ligands can be used for OTR and V1aR TR-FRET binding assay.

## Claims

1. A compound of formula (I): 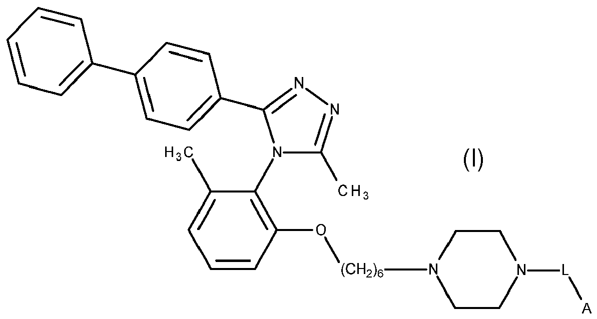 wherein L is a divalent linker and A is a functional group for coupling to a detectable labeling agent.

2. The compound according to claim 1, wherein L represents a divalent group chosen from: a substituted or unsubstituted, straight or branched chain, saturated or unsaturated C₁-C₁₀ alkylene moiety; a substituted or unsubstituted, saturated or unsaturated cycloalkylene moiety; a substituted or unsubstituted arylene moiety; a group of formula -CH₂CH₂(O-CH₂CH₂)ₙ- wherein n is an integer having a value from 1 to 5.

3. The compound according to claim 1 or 2, wherein A is a group chosen from: -NH₂, -NCS, -NCO, -N₃, -SH, -S-S-Ar or -NH-Ar, wherein Ar is a 5- or 6-membered, saturated or unsaturated heterocycle comprising 1 to 3 heteroatoms, said heterocycle being optionally substituted with a halogen atom, or A is chosen from one of the following groups:

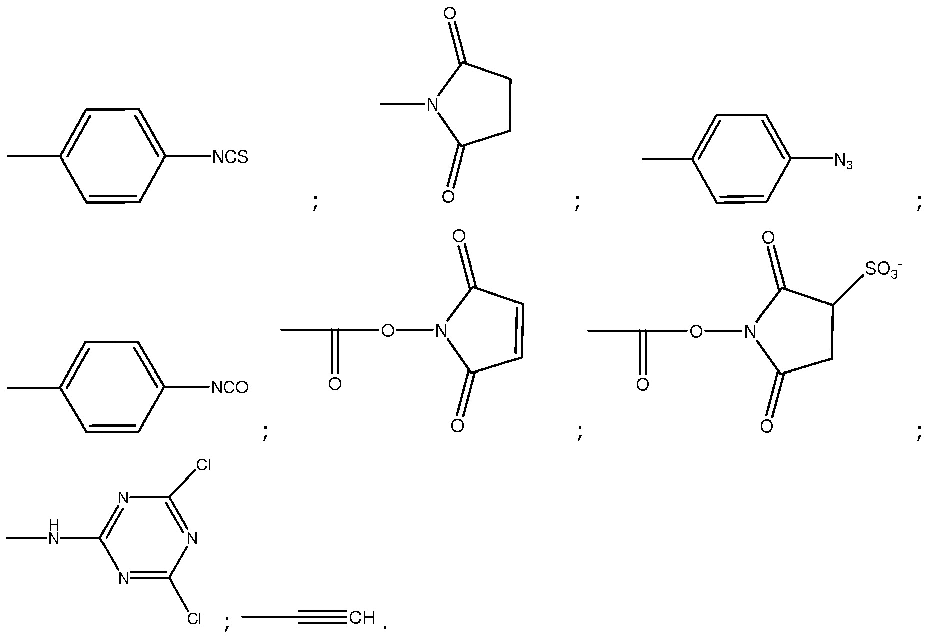

4. The compound according to any of the preceding claims, wherein L is a group of formula -CH₂CH₂(O-CH₂CH₂)ₙ- wherein n is as defined in claim 2, preferably n is 1, 2 or 3.

5. The compound according to any of the preceding claims, wherein A is -NH₂.

6. The compound according to any of the preceding claims, of formula (II): 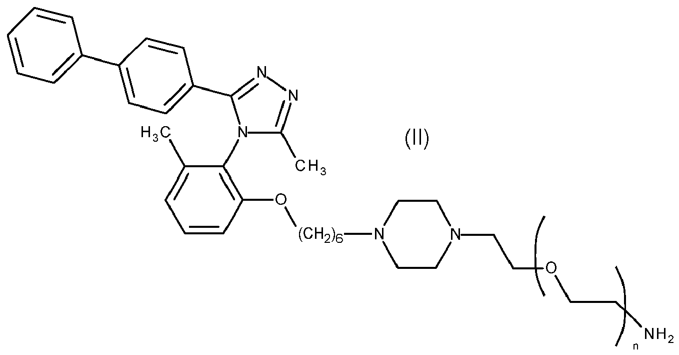 wherein n is an integer having a value from 1 to 5, preferably n is 1, 2 or 3.

7. A conjugate comprising the compound of any one of claims 1 to 6 in which a detectable labeling agent is coupled to group A of said compound.

8. The conjugate according to claim 7 in which the labeling agent is a luminescent substance.

9. The conjugate according to claim 8, wherein the luminescent substance is a fluorescent compound chosen from: rhodamines, cyanines, squaraines, fluoresceins, rare-earth chelates, rare-earth cryptates, quantum dots, phycobiliproteins, B-phycoerythrin, R-phycoerythrin, C-phycocyanin, and allophycocyanin.

10. The conjugate according to claim 9, wherein the fluorescent compound is Lumi4™Tb or Dy647.

11. Use of a conjugate according to any one of claims 7 to 10 in a binding assay for the oxytocin receptor or the vasopressin V1a receptor.

12. Use of a conjugate according to any one of claims 7 to 10 to label the oxytocin receptor or the vasopressin V1a receptor.

13. A method for assessing the capacity of a test compound to bind the oxytocin receptor or the vasopressin V1a receptor, which method comprises contacting said test compound with the oxytocin receptor or the vasopressin V1a receptor in the presence of a conjugate according to any one of claims 7 to 10, and measuring the signal generated by said conjugate.

14. The method according to claim 13, which further comprises comparing the signal generated by the conjugate with the signal measured in the absence of the test compound or with different concentrations of the test compound.
